# EUROPEAN PATENT APPLICATION

(11) **EP 4 287 209 A1**
(43) Date of publication of application: **06.12.2023**
(21) Application number: 22176811.2
(22) Date of filing: 01.06.2022
(51) Int. Cl.: G16H 50/20, A61B 5/103, A61B 5/11, G16H 50/70

(54) **METHOD AND DEVICE FOR NERVE FUNCTION ASSESSMENT**

(71) Applicant: Otto-von-Guericke-Universität Magdeburg, 39106 Magdeburg (DE)
(72) Inventor: MERTENS, Peter Rene, 39104 Magdeburg (DE); MING, Antao, 39120 Magdeburg (DE)

(57) **Abstract**

The invention relates to ways for determining, whether or not a person suffers from impaired nerve function. In order to make the generation of data for the determination easy for the person, the invention provides that sensor data from foot bottom pressure sensors (11, 12) used by the person to control computer games is used to determine, whether or not a person suffers from impaired nerve function.

## Description

### Field of the Invention

The invention generally relates to the assessment of nerve function of a person. In particular, the invention concerns a computer implemented method, a data processing device, a computer program and a system for indicating or determining, whether or not a person suffers from impaired nerve function.

### Technological Background

Impaired nerve function can lead to severe health issues which may lead to infection and may even require extremity amputation, in particular when the lower extremities are concerned. Yet, four out of five amputations may be prevented by adequate care and in case the impairment of the nerve function, particularly in the lower extremities, can be diagnosed in an early stage.

Impaired nerve function may be peripheral neuropathy, which may be caused by diabetes. Currently, according to the International Diabetes Federation, more than 435 million people worldwide are diagnosed with diabetes. This number is expected to rise to 693 million in 2045. However, diabetes may cause impaired nerve function and e.g. peripheral neuropathy even in a pre-diabetes stage, which is often not diagnosed prior to the diabetes presenting itself with symptoms. When diabetes symptoms are present, however, the impairment of the nerve function may already have led to further health issues.

Symptoms of peripheral neuropathy may commonly originate distally (e.g. at the tips of the toes) and spread proximally with a symmetric distribution. Peripheral nerves encompass Aδ- and C-fibers (small, spinothalamic; temperature sensation, nociception, generally assessed by pinprick), as well as Aβ-fibers (large, back nervous system; generally assessed by vibration and monofilament). In addition, the reflex status may be altered (Achilles tendon reflex impairment).

Known ways to indicate or determine, whether or not a person suffers from impaired nerve function, either require a medical professional to perform tests and expensive tools or lab tests, or are insufficiently sensitive overall or with respect to differently sized nerve fibers whose function may be impaired.

### Description of the Invention

In view of the above-mentioned disadvantages, an object of the invention may be to provide a way to indicate or determine, whether or not a person suffers from impaired nerve function, in an accurate, enjoyable, time-saving, yet cheap manner, which allows for the patient to perform the examination without having a medical professional performing the examination and without the need to use expensive tools or lab tests.

This object is achieved according to the invention in that the above-identified computer implemented method for indicating or determining, whether or not a person suffers from impaired nerve function, comprises loading sensor data from foot bottom pressure sensors that were pressed between the feet of the person and the underground while the person was playing a predetermined computer game while controlling the computer game by exerting pressure on the pressure sensors via the person's feet, and analyzing the sensor data with an algorithm that is adapted to detect whether or not the person suffers from impaired nerve function based at least on the sensor data.

For the above-identified data processing device with a storage device and a processor, the object may be achieved in that in the storage device comprises instructions, which, when executed by the processor cause the processor to carry out the computer implemented method for indicating or determining, whether or not a person suffers from impaired nerve function according to the invention.

For the above-identified computer program comprising instructions, the object may be achieved in that the instruction, when the program is executed by a computer, cause the computer to carry out the computer implemented method for indicating or determining, whether or not a person suffers from impaired nerve function according to the invention.

For the above-identified system, the object may be achieved in that the system comprises a gaming device for playing computer games, at least two foot bottom pressure sensors that are connectable to the gaming device in a control signal transmitting manner, and the data processing device according to the invention, wherein the data processing device is adapted to load the sensor data from foot bottom pressure sensors, from the gaming device or from another storage device of the system.

By indicating or determining, whether or not a person suffers from impaired nerve function, based on data from foot bottom pressure sensors used for controlling a game, the person can easily and even remotely generate the examination data, which allows for the indication or determination of possible nerve function impairment. No supervision of the person while playing the game is required. Pressure sensors per se and particularly when compared to known examination equipment are low cost.

The solution according to the invention can be further improved by the embodiments mentioned in the following, which, unless explicitly mentioned to the contrary, can be combined as desired. The embodiments and their possible advantages are elaborated below:
According to an embodiment, the sensor data and game data concerning the person's gaming performance are combined and analyzed by the algorithm.

An advantage of this embodiment may be that the impairment of the nerve function can be indicated or determined with higher accuracy, i.e. higher sensitivity and/or higher specificity.

According to an embodiment, the sensor data include data representing the amount of pressure exerted by the person and/or the timing of the pressure exerted by the person, optionally combined with game control timing achieved by the person, possibly in comparison to at least one preset threshold value for pressure amount and/or pressure timing.

An advantage of this embodiment may be that using data that represents different aspects of the pressure exerted by the person may further improve accuracy, i.e. sensitivity and/or specificity.

According to an embodiment, the game data include timing and/or accuracy of control of positioning an item of the game with respect to another item of the game.

An advantage of this embodiment may be that using data that represents different aspects of gaming performance of the person may further improve accuracy, i.e. sensitivity and/or specificity.

For example, data derived from the sensor data and/or the game data may be reaction time, sensation, skillfulness, muscle strength, balance, and/or endurance.

Particularly, if the sensor data and the game date is combined according to an embodiment, the accuracy of the indication or determination may be improved.

According to an embodiment, the algorithm is adapted to derive the person's capabilities from the sensor and optionally the game data, wherein the algorithm may be adapted to indicate or determine the person's balance, reaction time, skillfulness, endurance and/or muscle strength as the person's capabilities from the sensor data or the combination of sensor data and game data.

An advantage of this embodiment may be that derived not only raw data, but also derived capabilities of the person using the sensors to control the game can be used, which may further improve the accuracy of the indication or determination.

According to an embodiment, the algorithm is an artificial intelligence model trained to indicate or determine whether a person suffered from impaired nerve function based at least on the pressure data.

An advantage of this embodiment may be that parameters that were not thought to be indicative for impaired nerve function can be found during training of the artificial intelligence model and used by the trained artificial intelligence model. Alternatively, or additionally, the using the trained artificial intelligence model may further improve the accuracy of the indication or determination.

According to an embodiment, the computer game is a game of skill.

An advantage of this embodiment may be that games of skill not only provide a high motivation for playing, but further support by provoking the person who is playing the game to perform comparatively large and comparatively small movements with high and low forces, such that different fibers and reflexes are applied while playing the game of skill, which facilitates indication or determination of impaired function of differently sized fibers.

According to an embodiment, the system comprises shoe inlays or shoes, wherein the inlays of the inner sole of the shows each comprise at least one of the foot bottom pressure sensors. Alternatively, the foot bottom pressure sensors may be affixed to the person's foot bottoms, wherein this embodiment could require support by a third person for affixing the sensors. As a further alternative, the foot bottom pressure sensors may be provided in or at parts of socks that contact the foot bottoms of the person when the person is wearing the socks.

For example, pair of shoes harboring sensor-equipped insoles available by AntiSense System^{®}, IEE S.A. Luxembourg, may be used. These insoles each encompass an Electronic Control Unit (ECU) and e.g. eight pressure sensors (5,57 cm² high dynamic HD002 force sensing resistors) that may be integrated into the heel, lateral arch, metatarsal 1, 3, and 5, hallux, and toe region of the foot. The sensors may allow for pressure detection with a sensitivity of 3.4 mbar in the range between 250 mbar and 7 bar. The ECU may comprise a nine degrees of freedom inertial measuring unit (IMU, embedding a 3-axis accelerometer, a 3-axis gyroscope, and a 3-axis magnetometer), sampling rate up to 500Hz, data synchronization (between insoles and smart devices), automatic detection of foot side, internal storage of 16 Gigabytes, and up to 10 h energy supply. The sensors can be embedded in foil and may not protrude. The insole can be integrated into footwear with a support layer of ethylene-vinylacetat-30 material and a protective layer of sponge.

Sensor data can be recorded at more than 50 Hz, more than 100 Hz, up to or more than 200 Hz and transferred in real-time to the gaming device e.g. via Bluetooth or via cable for smooth steering of games. The person using the system for indicating or determining, whether or not the person suffers from impaired nerve function, may be seated on a chair without armrests in front of a table on which the gaming device, e.g. a tablet computer (for example Samsung Galaxy Tab A T580 or an iPad) can be positioned that connects to the insole via Bluetooth 5.0 or cable. The participants may initially calibrate the insole through eight predefined steps.

An advantage of this embodiment may be that no hardware needs to be specifically developed, but already available hardware can be used, which further reduces cost and increases availability.

A data processing device and/or a gaming device may be a computer with a processor, with a solid, non-transitory storage, with RAM, data interfaces like USB, HDMI Display port etc., and may in particular be any Personal Computer, desktop computer, laptop computer, tablet computer, smart phone, smart watch, server etc.

### Brief Description of the Drawings

The invention is described hereinafter in more detail and in an exemplary manner using advantageous embodiments and with reference to the drawings. The described embodiments are only possible configurations in which, however, the individual features as describes above can be provided independent of one another or can be omitted in the drawings:
For elements of an exemplary embodiment, which correspond in form and/or function to elements of another exemplary embodiment, the same reference numerals are used.
- Figure 1: shows an exemplary embodiment of a computer implemented method for indicating or determining, whether or not a person suffers from impaired nerve function, with steps that comprise gathering sensor data, which are not part of the claimed invention,
- Figure 2: shows an exemplary embodiment of a system for indicating or determining, whether or not a person suffers from impaired nerve function,
- Figures 3 to 7: show exemplary embodiments of games played by the person by means of foot bottom pressure sensors,
- Figure 8: shows an exemplary embodiment of a classification modeling scheme of an artificial intelligence, and
- Figure 9: schematically shows functionalities of an exemplary embodiment of the computer method implemented as the artificial intelligence.

### Description of Exemplary Embodiments

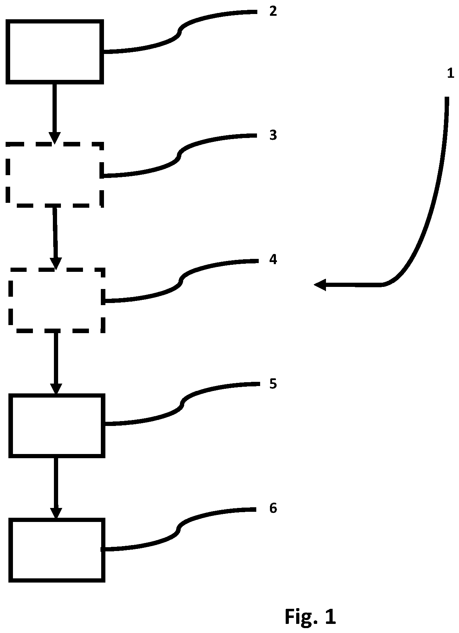

Figure 1 shows a first exemplary embodiment of a computer implemented method for indicating or determining, whether or not a person suffers from impaired nerve function, according to the invention and as a flow chart.

The computer implemented method 1 for indicating or determining, whether or not a person suffers from impaired nerve function, starts with a first method step 1. Method steps 3 and 4 concern bringing at least one foot bottom pressure sensor in contact with each of the person's feet (step 3) and to let the person play the games while gathering pressure and optional game data (step 4). Method steps 3 and 4 are not part of the claimed invention, but are useful for the understanding of the invention as claimed. Steps 3 and 4 may be supervised by another person, which may even assist the person that may have nerve function impairment, wherein the other person needs not to be of any medical profession.

In method step 5, the sensor data and optionally also the game data is analyzed with an algorithm that is adapted to detect whether or not the person suffers from impaired nerve function based on the sensor data.

Finally, the method 1 may end with method step 6, in which the results of the indication or determination may be communicated with the person or with another person, e.g. a medical practitioner usually examining or treating the person, like a general or specialized practitioner. The other person needs not to be present for applying the sensors or while playing the games.

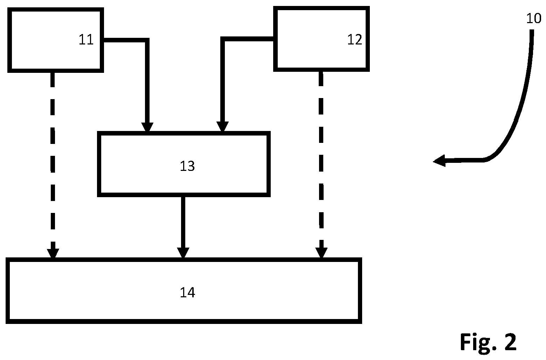

Figure 2 schematically shows an exemplary embodiment of the system for indicating or determining, whether or not a person suffers from impaired nerve function, according to the invention.

The system 10 comprises at least two foot bottom pressure sensors 11, 12, each of which being applied to the bottom of another foot of the person, such that the person can control games by differently pressing her or his feet on the ground. The system 10 further comprises a gaming device 13 for playing computer games. The foot bottom pressure sensors 11, 12 are connectable to the gaming device 13 in a control signal transmitting manner. The control signal depends and for example may be proportional to the pressure the person applies to the ground vis the foot bottom pressure sensors 11, 12. The control signal may be converted into sensor data by the foot bottom pressure sensors 11, 12 or by the gaming device 13. Optionally, the foot bottom pressure sensors 11, 12 may be connected in a signal and e.g. data transmitting manner to a data processing device 14 of the system 10. The data processing device 14 may, hence, be adapted to load the sensor data from foot bottom pressure sensors 11, 12, from the gaming device 13 or from another storage device of the system 10. The data processing device 14 may be the gaming device 13 or a part thereof, may be provided separate from the gaming device 13 and may be connected directly, e.g. wirelessly or via a cable, or via the internet to the gaming device 13 and/or the foot bottom pressure sensors 11, 12.

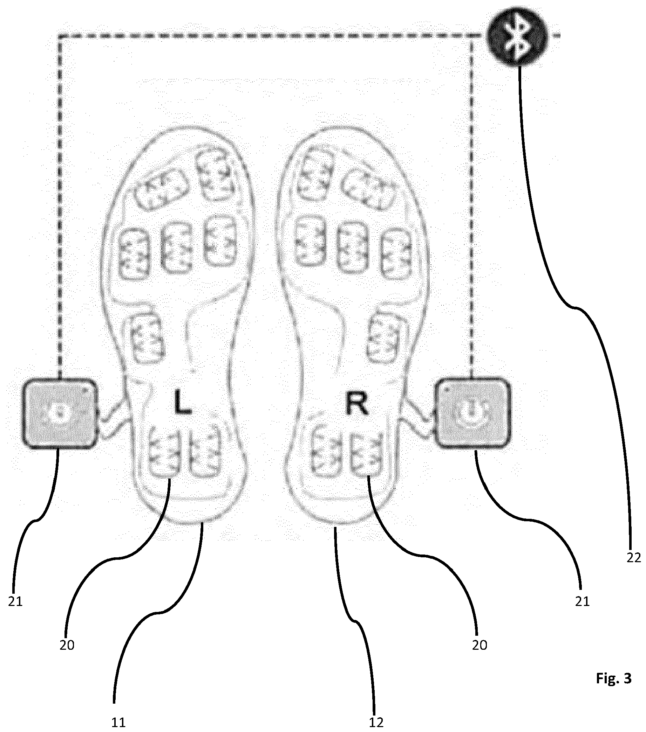

Figure 3 shows an exemplary embodiment of foot bottom pressure sensors 11, 12 in a schematic view.

The foot bottom pressure sensors 11, 12 may each comprise at least one or more than one pressure sensitive element 20, i.e. pressure sensor element. The foot bottom pressure sensors 11, 12 may be shoe inserts. The foot bottom pressure sensors 11, 12 may comprise a common signal to data converter 21, which is connected to the pressure sensitive elements 20 and converts the sensor signal into sensor data. The signal to data converter 21 may be an analog to digital converter. The exemplary embodiment of figure 3 shows the two foot bottom pressure sensors 11, 12, wherein each of the foot bottom pressure sensors 11, 12 comprises a signal to data converter 21. The foot bottom pressure sensors 11, 12 may a signal or data interface 22 that is adapted to output the sensor signal or the sensor data. The signal or data interface 22 may be a physical interface like a connector. The exemplary embodiment of figure 3 shows the two foot bottom pressure sensors 11, 12 with a wireless signal or data interface 22, which may output the sensor data wirelessly, e.g. via Bluetooth.

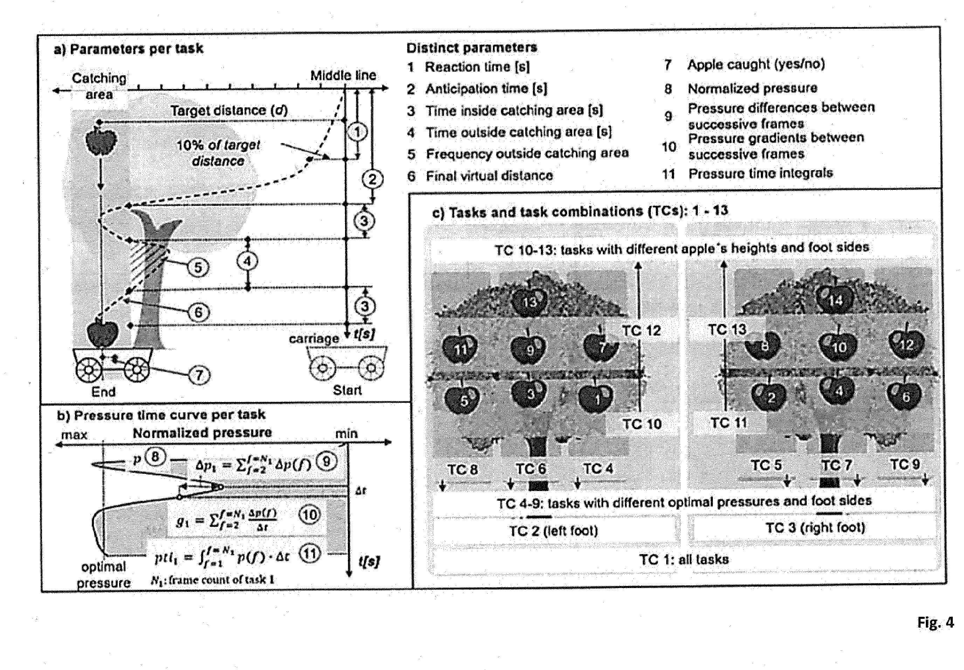

Figures 4 to 7 schematically depict games and how to control these games to generate the data, which, according to the invention, is used for indicating or determining, whether or not a person suffers from impaired nerve function.

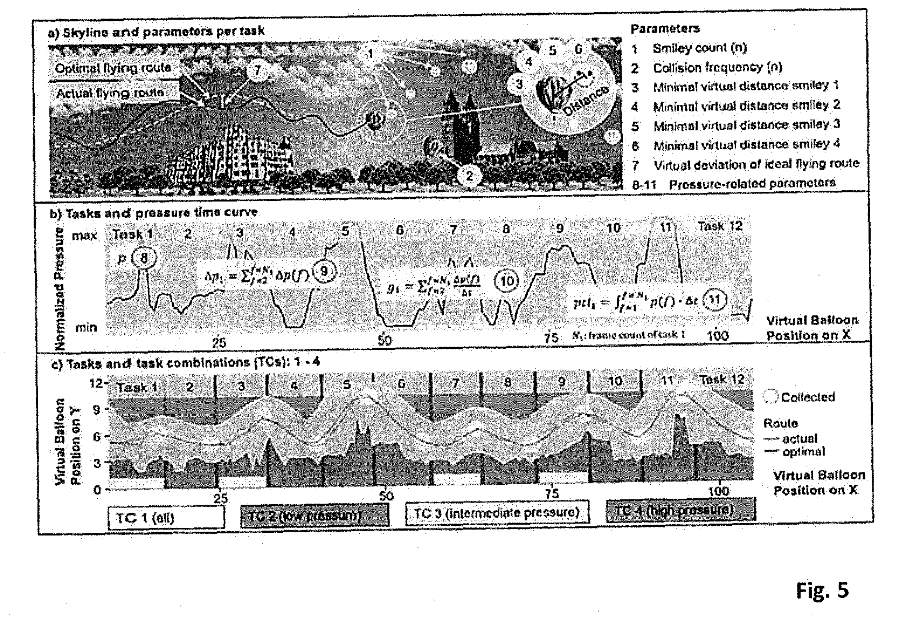

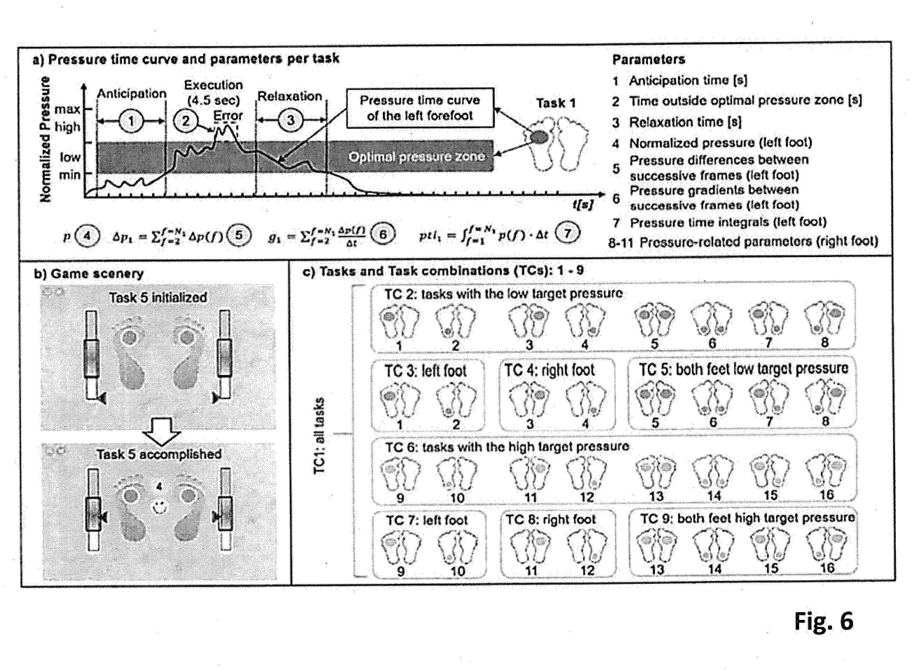

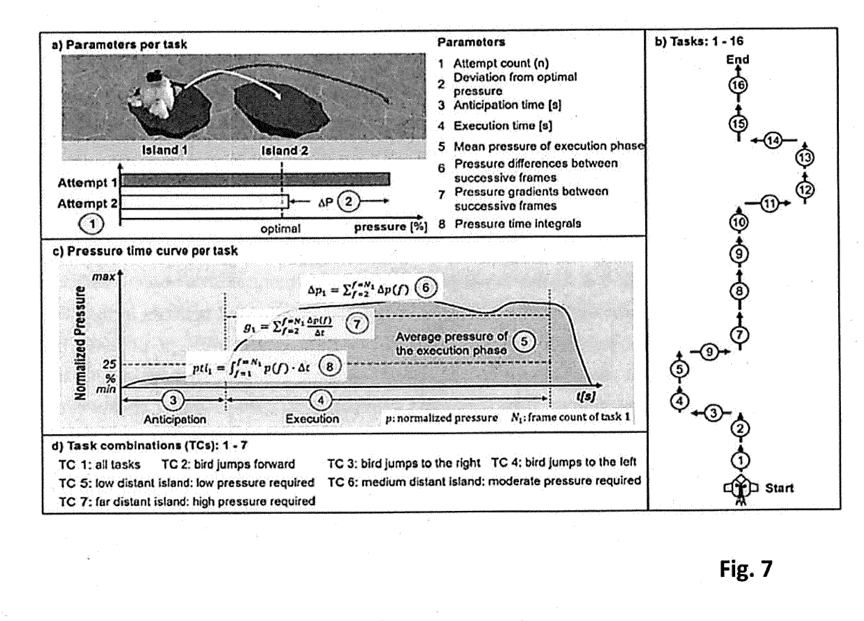

The measured pressure thresholds may be utilized to normalize the absolute pressure values, e.g. to the range of zero to one. Subsequently, the person may familiarize her- or himself with the setup and control techniques of the computer games through predefined tutorials that may be repeated on demand. Each gaming session may comprise a plurality of e.g. four games: Apple-Catch (AC, figure 4), Balloon- Flying (BF, figure 5), Cross-Pressure (CP figure 6), and Island-Jump (IJ, figure 7), which can be played selectively or sequentially. Each game may last several minutes, e.g. up to 5 minutes, up to 10 minutes, up to 15 minutes or about 20 minutes. The acquired sensor data and game data can transferred in a complete data package to a remote server for data visualization and analyses.

In the AC game, the person positions a carriage to catch apples falling from tree. The BF game challenged the person to fly a balloon along a predetermined path. The CP game required the person to provide predefined loads with predefined parts of the feet in predefined patterns. In the IJ game, the person controls an avatar to jump from one island to another island, wherein the at least one pressure variable like the amount of pressure of the timing of the pressure of the duration of the pressure determined whether the avatar lands on the other island. A known game with a similar approach but with a different control mechanism may be "Hot Lava" by Klei Entertainment Inc., which is controlled by game pads or tough screens, i.e. by hand. All movements of items (the carriage of the AC game, the balloon of the BF game, the avatar of the IJ game) and/or indicators on the CP game are controlled by the sensor signal, which depends from the person exerting pressure with his feet on the pressure sensors.

Game data extraction of distinct game parameters from defined game tasks and game task combinations for the games can be set up. Game parameters can be calculated from each game task and can be considered as primary features, such as reaction time e.g. in the first task of the AC game. Apart from that, the concept of task combination (TC) can be introduced, i.e., a set of game tasks with similar specifications or macro-measurements can be combined. For example, TC1 of the AC game can encompass all tasks of this game. TC2 can cover all tasks that can use the left foot for controlling the carriage movement to catch apples. The corresponding TC3 can consist of all tasks that use the right foot. The sum, mean, and standard deviation of primary features over game tasks of TCs can be treated as secondary features. For example, in the AC game, the reaction time of TC1 can be a secondary feature that can be computed from the average reaction time over all tasks. Overall, 1.880 distinctive parameters reflexing person's performance in the entirety and among similar tasks can be extracted per data set (per person and for all four games in total).

The games may provide sets of data that comprise the sensor data and optionally the game data, wherein the AC game may provide 583 data elements or features, the BF game may provide 528 data elements or features, the CJ game may provide 473 data elements or features, and the IJ game may provide 296 data elements or features, such that, in case the person plays all four games, in total 1880 data elements or features are present in the data set of sensor and optionally game data to indicate or determine, whether or not a person suffers from impaired nerve function.

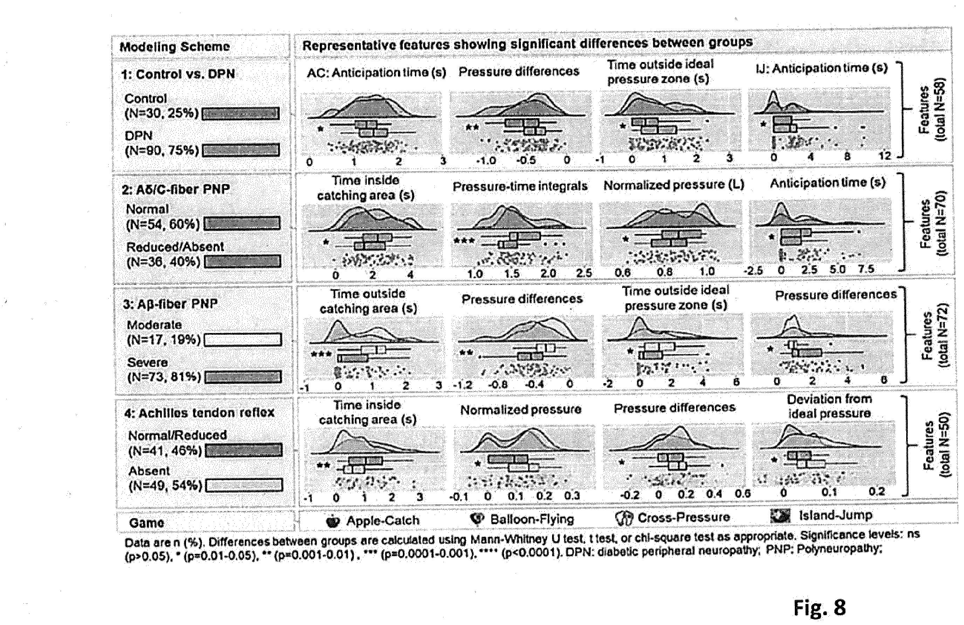

Figure 8 shows a classification scheme of an exemplary embodiment of an algorithm representing an Al (Artificial Intelligence) model for indicating or determining, whether or not a person suffers from impaired nerve function.

For example, Al (Artificial Intelligence) models can be derived to distinguish healthy controls and patients with diabetes and sensory neuropathy in an age-matched cohort (cohort 2) with acquired data sets. The NDS (neuropathy deficit score) can be utilized to generate class labels for supervised Al learning. NDS greater than or equal to 2 and impaired vibration sensation (0-4/8) can be considered as existing neuropathy (DPN), and NDS=0 can be considered as the absence of neuropathy (Control group). Secondly, in the DPN group, more detailed phenotyping can be achieved by analyzing the dysfunction of different fiber types. Figure 8 summarizes the detailed information of these modeling schemes. For model 2 of figure 8, severe damage of Aδ-/C-fibers can be assumed in patients with reduced/absent pinprick (nociception) or temperature sensation. For model 3 of figure 8, the presence of severe Aβ-fiber polyneuropathy can be assumed with impaired vibration sensation (below 3/8) or an abnormal 10g monofilament test result (reduced/absent). The remaining patients in the DPN group can be classified as moderate Aβ-fiber polyneuropathy (impaired vibration sensation: 3-4/8). For model 4 of figure 8, the absence of Achilles tendon reflexes can be considered as a positive label in the model training. A negative label may be assigned to normal or impaired reflexes.

For feature selection, acquired data sets can be initially split into testing and training data sets (e.g. ratio 3:7). The random sampling can occur in each class and preserved the overall class distribution of the data. Only the training data set can be imported to the classifier for estimating the importance of features depending on model-independent metrics, e.g., area under the receiver operating characteristic. Subsequently, models with different subsets of top-ranked features (i.e., variances of numbers and/or orders of features) can be tested. If the studied data set is imbalanced, Cohens Kappa (a classification accuracy normalized by the imbalance of the classes in the data) can be selected as the performance metric to compare the models trained with different feature combinations. The models with higher Cohens Kappa values can be chosen as candidate models.

Support vector machine (SVM) with three kernel functions (linear, radial, and polynomial) can be selected for model training. Clinical studies reported that SVM models perform better on medical classification issues with limited and imbalanced data sets. Three repeats five-fold cross-validation can be utilized in feature ranking and training to avoid overfitting and derive a more accurate estimate of the model performance. This statistical method can repeatedly divide the training dataset into e.g. five subsets with approximately equal size three times. Each subset can contain the same proportion of labels as the complete dataset. A subset and e.g. four of the five subsets can be utilized in the model training, while the remaining subset can be used for validation. The average accuracy of cross-validation can be considered in the grid search of parameters combination that improves the model performance the most. Ultimately, the obtained candidate models can be further applied to the testing data set to evaluate the models' predictive performance.

R programming language (version 4.0.4) and related open-source libraries can be utilized for statistical computing and machine learning algorithms.

For example, libraries that may be used are mentioned in the following (Package, Version, Description, Source, Published Date):
Caret, 6.0-86, misc functions for training and plotting classification and regression models, Comprehensive R Archive Network (CRAN), 2020-03-20 10:20:07 UTC
Colorspace, 2.0-0,"Carries out mapping between assorted color spaces including RGB, HSV, HLS, CIEXYZ, CIELUV, HCL (polar CIELUV), CIELAB, and polar CIELAB, qualitative, sequential, and diverging color palettes based on HCL colors are provided along with corresponding ggplot2 color scales, color palette choice is aided by an interactive app (with either a Tcl/Tk, or a shiny graphical user interface) and shiny apps with an HCL color picker and a color vision deficiency emulator. Plotting functions for displaying and assessing palettes include color swatches, visualizations of the HCL space, and trajectories in HCL and/or RGB spectrum. Color manipulation functions include: desaturation, lightening/darkening, mixing, and simulation of color vision deficiencies (deutanomaly, protanomaly, tritanomaly), Details can be found on the project web page at http://colorspace.R-Forge.R-proiect.org/ and in the accompanying scientific paper: Zeileis et al. (2020, Journal of Statistical Software, <doi:10.18637/jss.v096.i01>).", Comprehensive R Archive Network (CRAN), 2020-11-11 07:40:15 UTC
Corrplot, 0.84, "A graphical display of a correlation matrix or general matrix. It also contains some algorithms to do matrix reordering. In addition, corrplot is good at details, including choosing color, text labels, color labels, layout, etc.", Comprehensive R Archive Network (CRAN), 2017-10-16 22:58:08 UTC
Dfidx, 0.0-4, Provides extended data frames, with a special data frame column which contains two indexes, with potentially a nesting structure, Comprehensive R Archive Network (CRAN, 2021-02-03 05:40:10 UTC
Dplyr, 1.0.7, "A fast, consistent tool for working with data frame like objects, both in memory and out of memory.", Comprehensive R Archive Network (CRAN), 2021-06-18 23:20:01 UTC
Egg, 0.4.5, Miscellaneous functions to help customise 'ggplot2' objects. High-level functions are provided to post-process 'ggplot2' layouts and allow alignment between plot panels, as well as setting panel sizes to fixed values. Other functions include a custom 'geom', and helper functions to enforce symmetric scales or add tags to facetted plots, Comprehensive R Archive Network (CRAN), 2019-07-13 06:00:27 UTC
Formula, 1.2-4, "Infrastructure for extended formulas with multiple parts on the right-hand side and/or multiple responses on the left-hand side (see <doi:10.18637/jss.v034.i01>).", Comprehensive R Archive Network (CRAN), 2020-10-16 13:50:06 UTC
Gbm, 2.1.8, "An implementation of extensions to Freund and Schapire's AdaBoost algorithm and Friedman's gradient boosting machine. Includes regression methods for least squares, absolute loss, t-distribution loss, quantile regression, logistic, multinomial logistic, Poisson, Cox proportional hazards partial likelihood, AdaBoost exponential loss, Huberized hinge loss, and Learning to Rank measures (LambdaMart). Originally developed by Greg Ridgeway.", Comprehensive R Archive Network (CRAN), 2020-07-15 10:00:02 UTC
GGally, 2.1.0, "The R package 'ggplot2' is a plotting system based on the grammar of graphics. 'GGally' extends 'ggplot2' by adding several functions to reduce the complexity of combining geometric objects with transformed data. Some of these functions include a pairwise plot matrix, a two group pairwise plot matrix, a parallel coordinates plot, a survival plot, and several functions to plot networks.", Comprehensive R Archive Network (CRAN), 2021-01-06 01:20:06 UTC
Ggnewscale, 0.4.5, Use multiple fill and colour scales in 'ggplot2', Comprehensive R Archive Network (CRAN), 2021-01-11 08:00:35 UTC
ggplot2, 3.3.5 "A system for 'declaratively' creating graphics, based on ""The Grammar of Graphics"". You provide the data, tell 'ggplot2' how to map variables to aesthetics, what graphical primitives to use, and it takes care of the details.", Comprehensive R Archive Network (CRAN), 2021-06-25 11:30:09 UTC
ggpubr0.4.0, "The 'ggplot2' package is excellent and flexible for elegant data visualization in R. However the default generated plots requires some formatting before we can send them for publication. Furthermore, to customize a 'ggplot', the syntax is opaque and this raises the level of difficulty for researchers with no advanced R programming skills. 'ggpubr' provides some easy-to-use functions for creating and customizing 'ggplot2'- based publication ready plots.", Comprehensive R Archive Network (CRAN), 2020-06-27 06:20:02 UTC
ggrepel, 0.9.1, "Provides text and label geoms for 'ggplot2' that help to avoid overlapping text labels. Labels repel away from each other and away from the data points.", Comprehensive R Archive Network (CRAN), 2021-01-15 22:00:02 UTC
glmnet, 4.1, Extremely efficient procedures for fitting the entire lasso or elastic-net regularization path for linear regression, logistic and multinomial regression models, Poisson regression, Cox model, multiple-response Gaussian, and the grouped multinomial regression. There are two new and important additions. The family argument can be a GLM family object, which opens the door to any programmed family. This comes with a modest computational cost, so when the built-in families suffice, they should be used instead. The other novelty is the relax option, which refits each of the active sets in the path unpenalized. The algorithm uses cyclical coordinate descent in a path-wise fashion, as described in the papers listed in the URL below, Comprehensive R Archive Network (CRAN), 2021-01-11 08:00:30 UTC
gmodels, 2.18.1, Various R programming tools for model fitting, Comprehensive R Archive Network (CRAN), 2018-06-25 16:23:05 UTC
gridExtra, 2.3, "Provides a number of user-level functions to work with "grid" graphics, notably to arrange multiple grid-based plots on a page, and draw tables.", Comprehensive R Archive Network (CRAN), 2017-09-09 14:12:08 UTC
Hmisc, 4.4-2, "Contains many functions useful for data analysis, high-level graphics, utility operations, functions for computing sample size and power, importing and annotating datasets, imputing missing values, advanced table making, variable clustering, character string manipulation, conversion of R objects to LaTeX and html code, and recoding variables.", Comprehensive R Archive Network (CRAN), 2020-11-29 06:00:16 UTC
Imager, 0.42.3, "Fast image processing for images in up to 4 dimensions (two spatial dimensions, one time/depth dimension, one colour dimension). Provides most traditional image processing tools (filtering, morphology, transformations, etc.) as well as various functions for easily analysing image data using R. The package wraps 'Clmg', <http://cimg.eu>, a simple, modern C++ library for image processing.", Comprehensive R Archive Network (CRAN), 2020-05-11 16:40:06 UTC
Jpeg, 0.1-8.1, This package provides an easy and simple way to read, write and display bitmap images stored in the JPEG format. It can read and write both files and in-memory raw vectors., Comprehensive R Archive Network (CRAN), 2019-10-24 14:51:52 UTC
Knitr, 1.31, "Provides a general-purpose tool for dynamic report generation in R using Literate Programming techniques.", Comprehensive R Archive Network (CRAN), 2021-01-27 15:10:05 UTC
Lattice, 0.20-41, "A powerful and elegant high-level data visualization system inspired by Trellis graphics, with an emphasis on multivariate data. Lattice is sufficient for typical graphics needs, and is also flexible enough to handle most nonstandard requirements. See ?Lattice for an introduction.", Comprehensive R Archive Network (CRAN), 2020-04-02 12:00:06 UTC
Libcoin, 1.0-8, "Basic infrastructure for linear test statistics and permutation inference in the framework of Strasser and Weber (1999) <https://epub.wu.ac.at/102/>. This package must not be used by end-users. Comprehensive R Archive Network (CRAN) package 'coin' implements all user interfaces and is ready to be used by anyone.", Comprehensive R Archive Network (CRAN), 2021-02-08 11:00:03 UTC
Magrittr, 2.0.1, "Provides a mechanism for chaining commands with a new forward-pipe operator, %>%. This operator will forward a value, or the result of an expression, into the next function call/expression. There is flexible support for the type of right-hand side expressions. For more information, see package vignette. To quote Rene Magritte, "Ceci n'est pas un pipe."", Comprehensive R Archive Network (CRAN), 2020-11-17 16:20:06 UTC
Matrix, 1.3-2, "A rich hierarchy of matrix classes, including triangular, symmetric, and diagonal matrices, both dense and sparse and with pattern, logical and numeric entries. Numerous methods for and operations on these matrices, using 'LAPACK' and 'SuiteSparse' libraries.", Comprehensive R Archive Network (CRAN), 2021-01-06 17:40:19 UTC
Mboost, 2.9-4, "Functional gradient descent algorithm (boosting) for optimizing general risk functions utilizing component-wise (penalised) least squares estimates or regression trees as base-learners for fitting generalized linear, additive and interaction models to potentially high-dimensional data. Models and algorithms are described in \doi{10.1214/07-STS242}, a handson tutorial is available from \doi{10.1007/s00180-012-0382-5}. The package allows userspecified loss functions and base-learners.", Comprehensive R Archive Network (CRAN), 2020-12-10 09:40:02 UTC
MLeval, 0.3, Straightforward and detailed evaluation of machine learning models. 'MLeval' can produce receiver operating characteristic (ROC) curves, precision-recall (PR) curves, calibration curves, and PR gain curves. 'MLeval' accepts a data frame of class probabilities and ground truth labels, or, it can automatically interpret the Caret train function results from repeated cross validation, then select the best model and analyse the results. 'MLeval' produces a range of evaluation metrics with confidence intervals, Comprehensive R Archive Network (CRAN), 2020-02-12 06:40:02 UTC
Mlogit, 1.1-1, "Maximum likelihood estimation of random utility discrete choice models. The software is described in Croissant (2020) <doi:10.18637/jss.v095.i11> and the underlying methods in Train (2009) <doi:10.1017/CB09780511805271>.", Comprehensive R Archive Network (CRAN), 2020-10-02 12:12:05 UTC
Modeltools, 0.2-23, "A collection of tools to deal with statistical models. The functionality is experimental and the user interface is likely to change in the future. The documentation is rather terse, but packages 'coin' and 'party' have some working examples. However, if you find the implemented ideas interesting we would be very interested in a discussion of this proposal. Contributions are more than welcome!", Comprehensive R Archive Network (CRAN), 2020-03-05 11:50:06 UTC
Mvtnorm, 1.1-1, "Computes multivariate normal and t probabilities, quantiles, random deviates and densities.", Comprehensive R Archive Network (CRAN), 2020-06-09 15:50:02 UTC
NCmisc, 1.1.6, "A set of handy functions. Includes a versatile one line progress bar, one line function timer with detailed output, time delay function, text histogram, object preview, Comprehensive R Archive Network (CRAN) package search, simpler package installer, Linux command install check, a flexible Mode function, top function, simulation of correlated data, and more.", Comprehensive R Archive Network (CRAN), 2018-11-12 00:00:03 UTC
Party, 1.3-6, "A computational toolbox for recursive partitioning. The core of the package is ctree(), an implementation of conditional inference trees which embed tree-structured regression models into a well defined theory of conditional inference procedures. This nonparametric class of regression trees is applicable to all kinds of regression problems, including nominal, ordinal, numeric, censored as well as multivariate response variables and arbitrary measurement scales of the covariates. Based on conditional inference trees, cforest() provides an implementation of Breiman's random forests. The function mob() implements an algorithm for recursive partitioning based on parametric models (e.g. linear models, GLMs or survival regression) employing parameter instability tests for split selection. Extensible functionality for visualizing tree-structured regression models is available. The methods are described in Hothorn et al. (2006) <doi:10.1198/106186006X133933>, Zeileis et al. (2008) <doi:10.1198/106186008X319331> and Strobl et al. (2007) <doi:10.1186/1471-2105-8-25>.", Comprehensive R Archive Network (CRAN), 2021-02-08 12:20:02 UTC
Partykit, 1.2-12, "A toolkit with infrastructure for representing, summarizing, and visualizing tree-structured regression and classification models. This unified infrastructure can be used for reading/coercing tree models from different sources ('rpart', 'RWeka', 'PMML') yielding objects that share functionality for print()/plot()/predict() methods. Furthermore, new and improved reimplementations of conditional inference trees (ctree()) and model-based recursive partitioning (mob()) from the 'party' package are provided based on the new infrastructure. A description of this package was published by Hothorn and Zeileis (2015) <https://jmlr.org/papers/v16/hothorn15a.html>.", Comprehensive R Archive Network (CRAN), 2021-02-08 15:10:03 UTC
PerformanceAnalytics, 2.0.4, "Collection of econometric functions for performance and risk analysis. In addition to standard risk and performance metrics, this package aims to aid practitioners and researchers in utilizing the latest research in analysis of non-normal return streams. In general, it is most tested on return (rather than price) data on a regular scale, but most functions will work with irregular return data as well, and increasing numbers of functions will work with P&L or price data where possible.", Comprehensive R Archive Network (CRAN), 2020-02-06 12:10:11 UTC
plotROC, 2.2.1, "Most ROC curve plots obscure the cutoff values and inhibit interpretation and comparison of multiple curves. This attempts to address those shortcomings by providing plotting and interactive tools. Functions are provided to generate an interactive ROC curve plot for web use, and print versions. A Shiny application implementing the functions is also included.", Comprehensive R Archive Network (CRAN), 2018-06-23 07:52:56 UTC
plyr, 1.8.6, "A set of tools that solves a common set of problems: you need to break a big problem down into manageable pieces, operate on each piece and then put all the pieces back together. For example, you might want to fit a model to each spatial location or time point in your study, summarise data by panels or collapse high-dimensional arrays to simpler summary statistics. The development of 'plyr' has been generously supported by 'Becton Dickinson'.", Comprehensive R Archive Network (CRAN), 2020-03-03 14:40:02 UTC
png, 0.1-7, This package provides an easy and simple way to read, write and display bitmap images stored in the PNG format. It can read and write both files and in-memory raw vectors., Comprehensive R Archive Network (CRAN), 2013-12-03 22:25:05
pROC, 1.17.0.1, Tools for visualizing, smoothing and comparing receiver operating characteristic (ROC curves). (Partial) area under the curve (AUC) can be compared with statistical tests based on U-statistics or bootstrap. Confidence intervals can be computed for (p)AUC or ROC curves, Comprehensive R Archive Network (CRAN), 2021-01-13 14:30:02 UTC
psych, 2.0.12, A general purpose toolbox for personality, psychometric theory and experimental psychology. Functions are primarily for multivariate analysis and scale construction using factor analysis, principal component analysis, cluster analysis and reliability analysis, although others provide basic descriptive statistics. Item Response Theory is done using factor analysis of tetrachoric and polychoric correlations. Functions for analyzing data at multiple levels include within and between group statistics, including correlations and factor analysis. Functions for simulating and testing particular item and test structures are included. Several functions serve as a useful front end for structural equation modeling. Graphical displays of path diagrams, factor analysis and structural equation models are created using basic graphics. Some of the functions are written to support a book on psychometric theory as well as publications in personality research. For more information, see the <https://personalityproject.org/r/> web page, Comprehensive R Archive Network (CRAN), 2020-12-16 16:10:03 UTC
readxl, 1.3.1, "Import excel files into R. Supports '.xls' via the embedded 'libxls' C library <https://github.com/libxls/libxls> and '.xlsx' via the embedded 'RapidXML' C++ library <http://rapidxml.sourceforge.net>. Works on Windows, Mac and Linux without external dependencies.", Comprehensive R Archive Network (CRAN), 2019-03-13 16:30:02 UTC
reshape2, 1.4.4, "Flexibly restructure and aggregate data using just two functions: melt and 'dcast' (or 'acast').", Comprehensive R Archive Network (CRAN), 2020-04-09 13:50:02 UTC
ROCR, 1.0-11, "ROC graphs, sensitivity/specificity curves, lift charts, and precision/recall plots are popular examples of trade-off visualizations for specific pairs of performance measures. ROCR is a flexible tool for creating cutoff-parameterized 2D performance curves by freely combining two from over 25 performance measures (new performance measures can be added using a standard interface). Curves from different cross-validation or bootstrapping runs can be averaged by different methods, and standard deviations, standard errors or box plots can be used to visualize the variability across the runs. The parameterization can be visualized by printing cutoff values at the corresponding curve positions, or by coloring the curve according to cutoff. All components of a performance plot can be quickly adjusted using a flexible parameter dispatching mechanism. Despite its flexibility, ROCR is easy to use, with only three commands and reasonable default values for all optional parameters.", Comprehensive R Archive Network (CRAN), 2020-05-02 14:50:05 UTC
Rpart, 4.1-15, "Recursive partitioning for classification, regression and survival trees. An implementation of most of the functionality of the 1984 book by Breiman, Friedman, Olshen and Stone.", Comprehensive R Archive Network (CRAN), 2019-04-12 14:32:39 UTC
Sandwich, 3.0-0, "Object-oriented software for model-robust covariance matrix estimators. Starting out from the basic robust Eicker-Huber-White sandwich covariance methods include: heteroscedasticity-consistent (HC) covariances for cross-section data; heteroscedasticity- and autocorrelation-consistent (HAC) covariances for time series data (such as Andrews' kernel HAC, Newey-West, and WEAVE estimators); clustered covariances (one-way and multi-way); panel and panel-corrected covariances; outer-product-of-gradients covariances; and (clustered) bootstrap covariances. All methods are applicable to (generalized) linear model objects fitted by lm() and glm() but can also be adapted to other classes through S3 methods.
Details can be found in Zeileis et al. (2020) <doi:10.18637/jss.v095.i01>, Zeileis (2004) <doi:10.18637/jss.v011.i10> and Zeileis (2006) <doi:10.18637/jss.v016.i09>.", Comprehensive R Archive Network (CRAN), 2020-10-02 10:40:02 UTC
Stabs, 0.6-4, "Resampling procedures to assess the stability of selected variables with additional finite sample error control for high-dimensional variable selection procedures such as Lasso or boosting. Both, standard stability selection (Meinshausen & Buhlmann, 2010, <doi:10.1111/j.1467-9868.2010.00740.x>) and complementary pairs stability selection with improved error bounds (Shah & Samworth, 2013, <doi:10.1111/j.1467-9868.2011.01034.x>) are implemented. The package can be combined with arbitrary user specified variable selection approaches.", Comprehensive R Archive Network (CRAN), 2021-01-29 10:00:02 UTC
Strucchange, 1.5-2, "Testing, monitoring and dating structural changes in (linear) regression models. strucchange features tests/methods from the generalized fluctuation test framework as well as from the F test (Chow test) framework. This includes methods to fit, plot and test fluctuation processes (e.g., CUSUM, MOSUM, recursive/moving estimates) and F statistics, respectively. It is possible to monitor incoming data online using fluctuation processes. Finally, the breakpoints in regression models with structural changes can be estimated together with confidence intervals. Emphasis is always given to methods for visualizing the data.", Comprehensive R Archive Network (CRAN), 2019-10-12 18:35:49 UTC
Survival, 3.2-7, "Contains the core survival analysis routines, including definition of Surv objects, Kaplan-Meier and Aalen-Johansen (multi-state) curves, Cox models, and parametric accelerated failure time models.", Comprehensive R Archive Network (CRAN), 2020-09-28 08:20:02 UTC
VIM, 6.1.0, "New tools for the visualization of missing and/or imputed values are introduced, which can be used for exploring the data and the structure of the missing and/or imputed values. Depending on this structure of the missing values, the corresponding methods may help to identify the mechanism generating the missing values and allows to explore the data including missing values. In addition, the quality of imputation can be visually explored using various univariate, bivariate, multiple and multivariate plot methods. A graphical user interface available in the separate package VIMGUI allows an easy handling of the implemented plot methods.", Comprehensive R Archive Network (CRAN), 2021-01-19 17:00:13 UTC
Xlsx, 0.6.5, Provide R functions to read/write/format Excel 2007 and Excel 97/2000/XP/2003 file formats, Comprehensive R Archive Network (CRAN), 2020-11-10 15:00:02 UTC
Xts, 0.12.1, Provide for uniform handling of R's different time-based data classes by extending zoo, maximizing native format information preservation and allowing for user level customization and extension, while simplifying cross-class interoperability, Comprehensive R Archive Network (CRAN), 2020-09-09 16:40:03 UTC
Zoo, 1.8-8, "An S3 class with methods for totally ordered indexed observations. It is particularly aimed at irregular time series of numeric vectors/matrices and factors. zoo's key design goals are independence of a particular index/date/time class and consistency with ts and base R by providing methods to extend standard generics.", Comprehensive R Archive Network (CRAN), 2020-05-02 16:14:00 UTC

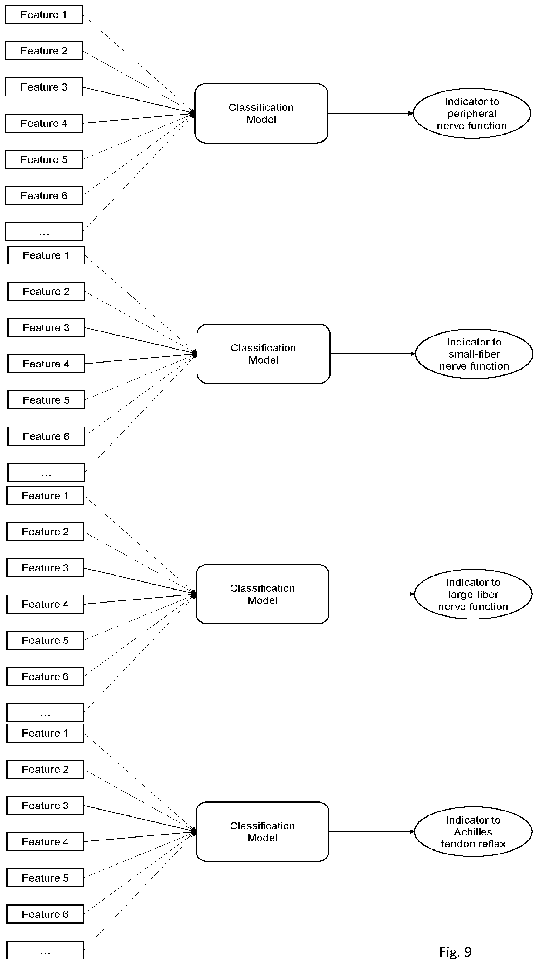

Figure 9 schematically shows, how the computer implemented method, as the artificial intelligence model, functions.

As presented in the figure 9, the inputs can be extracted features/parameters from sensor data and game results. Output can be a predictive indicator of possibility that the person suffers from impairment of differently sized nerves. The classification model can save the data patterns of healthy subjects and individuals with nerve impairment, which are learnt from previous acquired datasets. The workflow can be that the model tries to draw a conclusion on the possibility that the person suffers from impaired nerve function from inputted values. This calculation process can happens in the data process device 14. The applied models to learn the data patterns can be support vector machine (SVM), random forest (RF), artificial neural network (ANN), etc.

### Reference Signs

- 1: method
- 2: start
- 3: bring feet in contact with sensors
- 4: play game and gather data
- 5: analyze sensor data and indicate or determine nerve function impairment
- 6: end
- 10: system
- 11,12: foot bottom pressure sensors
- 13: gaming device
- 14: data processing device
- 20: pressure sensitive element
- 21: signal to data converter
- 22: data interface

## Claims

1. A computer implemented method (1) for indicating, whether or not a person suffers from impaired nerve function,
**characterized in that** the method (1) comprises
loading sensor data from foot bottom pressure sensors (11, 12) that were pressed between the feet of the person and the underground while the person was playing a predetermined computer game while controlling the computer game by exerting pressure on the pressure sensors via the person's feet, and
analyzing (5) the sensor data with an algorithm that is adapted to detect whether or not the person suffers from impaired nerve function based on the sensor data.

2. The method (1) of claim 1, **characterized in that** the sensor data and game data concerning the person's gaming performance are combined and analyzed (5) by the algorithm.

3. The method (1) of claim 1 or 2, **characterized in that** the sensor data include data representing the amount of pressure exerted by the person and/or the timing of the pressure exerted by the person.

4. The method (1) of claim 2 or 3, **characterized in that** the game data include timing and/or accuracy of control of positioning an item of the game with respect to another item of the game.

5. The method (1) of any one of claims 1 to 4, **characterized in that** the algorithm is adapted to indicate the person's balance, reaction time, skillfulness, endurance and/or muscle strength from the sensor data or the combination of sensor data and game data.

6. The method (1) of any one of claims 1 to 5, **characterized in that** the algorithm is an artificial intelligence model trained to indicate whether a person suffered from impaired nerve function based at least on the pressure data.

7. A data processing device (14) with a storage device and a processor, wherein the storage device comprises instruction, which, when executed by the processor cause the processor to carry out the method of any of claims 1 to 6.

8. A computer program comprising instructions, which, when the program is executed by a computer, cause the computer to carry out the method (1) of any of claims 1 to 6.

9. A system (10) for indicating, whether or not a person suffers from impaired nerve function,
**characterized in that**
the system comprises a gaming device (13) for playing computer games,
at least two foot bottom pressure sensors (11, 12) that are connectable to the gaming device (13) in a control signal transmitting manner, and
the data processing device (14) of claim 7, wherein
the data processing device (14) is adapted to load the sensor data from foot bottom pressure sensors (11, 12), from the gaming device (13) or from another storage device of the system.
